# EUROPÄISCHE PATENTANMELDUNG

(11) **EP 2 033 621 A2**
(43) Veröffentlichungstag der Anmeldung: **11.03.2009**
(21) Anmeldenummer: 08105072.6
(22) Anmeldetag: 19.08.2008
(51) Int. Cl.: A61K 8/31, A61K 8/34, A61K 8/37, A61Q 9/02

(54) **Verminderung des Verklebens von Rasierscherblättern**

(30) Priorität: 30.08.2007 DE 102007041863
(71) Anmelder: Beiersdorf AG, 20253 Hamburg (DE)
(72) Erfinder: zu Putlitz, Corinna, 25474, Bönningstedt (DE); Treu, Jens, 22844, Norderstedt (DE); Hülshoff, Anke, 22529, Hamburg (DE); Kauffeldt, Martin, 22303, Hamburg (DE); Kröpke, Rainer, 22869, Schenefeld (DE)

(57) **Zusammenfassung**

Die vorliegende Erfindung betrifft die Verminderung des Verklebens der Rasierscherblätter von Elektrorasierern durch Rasierhilfszubereitungen die spezielle Zusätze enthalten.

## Beschreibung

Die vorliegende Erfindung betrifft die Verminderung des Verklebens der Rasierscherblätter von Elektrorasierern durch Rasierhilfszubereitungen die spezielle Zusätze enthalten.

Schon seit Jahren werden neben der eigentlichen Trockenrasur mittels Elektrorasierer zunehmend Elektrorasierer verwendet, die zusammen mit einer Rasierhilfszubereitung angewendet werden. Ein Rasierer, wie er in der europäischen Patentanmeldung WO 98/08659 beschrieben ist, stellt eine Zwischenform zwischen Nass- und Trockenenrasierer dar. Er weist wie ein klassischer Trockenrasierer ein Scherblatt auf, zusätzlich kann aber eine Rasierhilfszubereitung dosiert ausgebracht werden, so dass während der Rasur die Haut mit der Rasierhilfszubereitung in Kontakt gebracht wird. Die Rasierhilfszubereitungen unterstützen die Gleiteigenschaften eines Trockenrasierers. Um ein gutes Ergebnis zu erhalten, kommt es u. a. darauf an, dass der Gleitfilm nicht zu dick oder zu zäh ist, und es nicht zu einer Schaumbildung kommt, die den Abstand zwischen Haut und Rasierer vergrößert.

In DE 103 36 044 wird eine Rasierhilfszubereitungen beschrieben, die die Korrosion eines Rasierers vermindert bzw. verhindert. Nachteilig bei der Anwendung der elektrischen Nassrasur ist jedoch, das die Zubereitungsbestandteile zusammen mit Barthaarresten und abgelösten Hautschuppen zur Verklebung der Scherblätter führen. Ein weiteres Problem ist das Verkleben des Rasierers selbst nach Beendigung der Rasur, wenn nicht eine sofortige Reinigung erfolgt. Diese Verklebungen wirken sich nachteilig auf die Rasierleistung aus. Elektronaßrasierer müssen daher nach jeder Rasur umständlich und aufwendig gereinigt werden. Eine unsachgemäße Reinigung birgt die Gefahr der Beschädigung der filigranen Scherblätter in sich bzw. führt dazu dass die Scherblätter sehr schnell an Schärfe verlieren.

Diesem Nachteil des Standes der Technik galt es also abzuhelfen.

Für den Fachmann unerwartet war es, das bestimmte Zusatzstoffe in Kombination mit einem Kohlenwasserstoff die Verklebung der Scherblätter verhindern bzw. vermindern können.

Rasierhilfszubereitungen können als Emulsion (lipid- und emulgatorhaltig), Gelcreme (lipidhaltig und emulgatorfrei) oder als Gel (lipid- und emulgatorfrei) vorliegen und sind bereits vielfach im Stand der Technik beschrieben.

Erfindungsgemäße Rasierhilfszubereitungen weisen neben den üblichen Inhaltsstoffen mindestens ein oder mehrere Substanzen gewählt aus der Gruppe (A) und mindestens einen Kohlenwasserstoff mit einer Kohlenstoffatomanzahl von 8 bis 22

Die Gruppe (A) umfasst: 1,3,4,6,7,8-Hexahydro-4,6,6,7,8,8-hexamethylcyclopenta-gamma-2-benzopyran, 2,6-Dimethyl-7-octen-2-ol, 2-Acetonapthone-1,2,3,4,5,6,7,8-octahydro-2,3,8,8-tetramethyl, 2-Isobutyl-4-hydroxy-4-methyltetrahydropyran, 2-tert-Pentylcyclohexylacetat, 3,7-Dimethyl-2,6-octadien-1-ol, 3-Methyl-5-phenyl-1-pentanol, 7-Acetyl-1,1,3,4,4,6-hexamethyltetralin, Adipinsäurediester, alpha-Amylcinnamaldehyd, alpha-Isomethylionon, Alpha-Methylionon, Amyl C Butylphenylmethylpropionalcinnamal, Amylcinnamylalkohol, Amylsalicylat, Anisalkohol, Benzoin, Benzylacetat, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citral, Citronellol, Citronellylmethylcrotonat, Citronenöl, Coumarin, Diethylsuccinat, d-Limonene, Ethylenbrassylate, Ethyllinalool, Eugenol, Evernia Furfuracea Extract, Evernia Prunastri Extract, Farnesol, Geraniol, Guajakholzöl, Heliotropin, Hexylcinnamal, Hexylsalicylat, Hydroxycitronellal, Hydroxyisohexyl 3-Cyclohexencarboxaldehyde, Isoeugenol, Lavendelöl, Lemonenöl, Limonen, Linalool, Linalylacetat, Mandarinenöl, Menthyl PCA, Methyl-2 Octynoat, Methylbenzoat, BHT, Methylcedrylketone, Methyldihydrojasmonate, Methylheptenon, Muskatnussöl, p-t-Butyl-alpha-methyldihydrocinnamic aldehyde, Rosmarinöl, süßes Orangenöl, Terpineol, Tonkabohnenöl, Triethylcitrat, Vanillin.

Erfindungsgemäß handelt es sich bei den Kohlenwasserstoffen bevorzugt um geradkettige oder verzeigte Kohlenwasserstoffe.

Diese Kombination aus unpolaren Kohlenwasserstofflipid und polaren Substanzen bewirkt, dass Bestandteile auf den Scherköpfen emulgieren. Unter laufenden, warmen Wasser, lassen sich dann die Scherköpfe leicht reinigen.

Vorteilhaft ist es mindestens zwei unterschiedliche Kohlenwasserstoffe in Kombination mit einer Substanz aus der oben genannten Gruppe einzusetzen bzw. einen Kohlenwasserstoff mit zwei Substanzen aus der oben genannten Gruppe einzusetzen, so dass sich eine Dreierkombination ergibt.

Als vorteilhaft hat sich herausgestellt, wenn mindestens ein Kohlenwasserstoff mit einer Kohlenstoffatomanzahl von 10 bis 20 Kohlenstoffatomen, insbesondere 12 bis 18 Kohlenstoffatomen, ganz besonders 13 bis 16 Kohlenstoffatomen eingesetzt wird.

Besonders vorteilhaft ist es, wenn als Kohlenwasserstoff Isohexadekan und/oder C13-C16 Isoparaffin gewählt werden.

Erfindungsgemäß vorteilhaft ist es, mindestens eine der folgenden Substanzen der Gruppe (A) einzusetzen: Linalool, Limonen, Benzylsalicylat, Hydroxyisohexyl 3-Cyclohexencarboxaldehyde, Isoeugenol, Hydroxycitronellal, Coumarin, Eugenol, Citral, BHT, Citronellol, Geraniol,Benzylbenzoate, Cinnamal, Benzylalkohol, Farnesol, Cinnamy-Ialkohol.

Zubereitungen der erfinderischen Zusammensetzung weisen gegenüber dem Stand der Technik zudem verbesserte Gleiteigenschaften auf, so dass die Rasur schneller und gründlicher wird. Wenn die Scherblätter besser über die Haut gleiten können, so ist auch das Irritationspotential beim Rasieren geringer. Das heißt, die Haut reagiert nicht mit starker Rötung oder Mikroverletzungen, was im Allgemeinen als sanftere Rasur bezeichnet wird.

Erfindungsgemäß besonders Vorteilhaft ist es, wenn die Rasierhilfszubereitung als O/W-Emulsion formuliert wird.

Die Rasierhilfszubereitungen können selbstverständlich noch weitere für kosmetische Produkte übliche Inhaltstoffe wie Tenside, Emollients, Konservierungsmittel, Moisturizer, Lipide enthalten.

Es folgen vorteilhafte Ausführungsbeispiele der vorliegenden Erfindung.

### O/W-Emulsion

| | **1** | **2** | **3** | **4** | **5** |
|---|---|---|---|---|---|
| lycerylstearatcitrat | 1,0 | 0,5 | --- | 1,5 | 0,3 |
| Linalool | 0,02 | 0,5 | 0,02 | 0,125 | 0,035 |
| Limonen | 0,01 | 0,025 | 0,01 | 0,125 | 0,035 |
| Benzylsalicylat | 0,0014 | 0,0065 | 0,0076 | 0,105 | 0,0088 |
| Vitamin-E-Acetat | 0,1 | 1,25 | 0,1 | 1,0 | 0,75 |
| C13-16 Isoparaffin | 1 | --- | 2,0 | 1,2 | 0,2 |
| Isohexadecan | --- | 2,0 | --- | --- | --- |
| Kamillenextrakt | 0,1 | 0,01 | 0,001 | 0,25 | 0,05 |
| Sojaöl | 1,0 | 0,1 | 0,9 | 2,5 | 0,5 |
| Xanthan Gum | 0,5 | 0,5 | 0,2 | 0,25 | 0,1 |
| Acrylat/C10-30 Alkylacrylat Crosspolymer | 0,5 | 0,25 | 0,25 | 0,15 | 0,65 |
| Panthenol 75%ig | 0,5 | 1,0 | 0,3 | 0,375 | 1,33 |
| PEG 45 M | 0,55 | 0,35 | 0,375 | 0,1 | 1,0 |
| Parfum | 0,3 | 0,25 | 0,18 | 0,375 | 0,775 |
| Methylparaben | 0,1 | 0,3 | 0,3 | 0,3 | 0,1 |
| Propylparaben | 0,3 | 0,25 | --- | 0,15 | --- |
| Methylisothiazolinone | 0,05 | --- | --- | --- | 0,1 |
| Phenoxyethanol | 1,0 | 0,5 | 0,6 | 0,15 | 0,8 |
| Sorbitol | 2,75 | --- | -- | 2,4 | --- |
| Butylenglykol | --- | --- | --- | 5 | 10 |
| Propylenglykol | --- | --- | 2,6 | 2,6 | --- |
| Glycerin | --- | 7,5 | --- | --- | --- |
| Wasser | ad 100 | ad 100 | ad 100 | ad 100 | ad 100 |

## Patentansprüche

1. Rasierhilfszubereitung enthaltend mindestens eine Substanz gewählt aus der Gruppe (A) und mindestens einen Kohlenwasserstoff, **dadurch gekennzeichnet, dass** die Gruppe (A) folgende Substanzen umfasst: 1,3,4,6,7,8-Hexahydro-4,6,6,7,8,8-hexamethylcyclopenta-gamma-2-benzopyran, 2,6-Dimethyl-7-octen-2-ol, 2-Acetonapthone-1,2,3,4,5,6,7,8-octahydro-2,3,8,8-tetramethyl, 2-Isobutyl-4-hydroxy-4-methyltetrahydropyran, 2-tert-Pentylcyclohexylacetat, 3,7-Dimethyl-2,6-octadien-1-ol, 3-Methyl-5-phenyl-1-pentanol, 7-Acetyl-1,1,3,4,4,6-hexamethyltetralin, Adipinsäurediester, alpha-Amylcinnamaldehyd, alpha-Isomethylionon, Alpha-Methylionon, Amyl C Butylphenylmethylpropionalcinnamal, Amylcinnamylalkohol, Amylsalicylat, Anisalkohol, Benzoin, Benzylacetat, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citral, Citronellol, Citronellylmethylcrotonat, Citronenöl, Coumarin, Diethylsuccinat, d-Limonene, Ethylenbrassylate, Ethyllinalool, Eugenol, Evernia Furfuracea Extract, Evernia Prunastri Extract, Farnesol, Geraniol, Guajakholzöl, Heliotropin, Hexylcinnamal, Hexylsalicylat, Hydroxycitronellal, Hydroxyisohexyl 3-Cyclohexencarboxaldehyde, Isoeugenol, Lavendelöl, Lemonenöl, Limonen, Linalool, Linalylacetat, Mandarinenöl, Menthyl PCA, Methyl-2 Octynoat, Methylbenzoat, Methylcedrylketone, Methyldihydrojasmonate, Methylheptenon, Muskatnussöl, p-t-Butyl-alpha-methyldihydrocinnamic aldehyde, Rosmarinöl, süßes Orangenöl, Terpineol, Tonkabohnenöl, Triethylcitrat, Vanillin und wobei der Kohlenwasserstoff 8 bis 22 Kohlenstoffatome aufweist.

2. Rasierhilfszubereitung nach Anspruch 1, **dadurch gekennzeichnet, dass** mindestens ein Kohlenwasserstoff geradkettiger und/oder verzweigter Natur ist.

3. Rasierhilfszubereitung nach Anspruch 2, **dadurch gekennzeichnet, dass** mindestens ein Kohlenwasserstoff 10 bis 20 Kohlenstoffatomen aufweist, insbesondere 12 bis 18 Kohlenstoffatomen, ganz besonders 13 bis 16 Kohlenstoffatomen.

4. Rasierhilfszubereitung nach Anspruch 3, **dadurch gekennzeichnet, dass** als Kohlenwasserstoff Isohexadekan und/oder C13- C16 Isoparaffin gewählt wird.

5. Rasierhilfszubereitung nach mindestens einem der vorhergehenden Ansprüche, **dadurch gekennzeichnet, dass** die Substanz der Gruppe (A) gewählt wird aus: Linalool, Limonen, Benzylsalicylat, Hydroxyisohexyl 3-Cyclohexencarboxaldehyde, Isoeugenol, Hydroxycitronellal, Coumarin, Eugenol, Citral, BHT, Citronellol, Geraniol,Benzylbenzoate, Cinnamal, Benzylalkohol, Farnesol, Cinnamylalkohol.

6. Rasierhilfszubereitungen, nach Anspruch 5, **dadurch gekennzeichnet, dass** die Substanz der Gruppe (A) Linalool ist.

7. Rasierhilfszubereitung, nach mindestens einem der vorhergehenden Ansprüche, **dadurch gekennzeichnet, dass** sie einem Kohlenwasserstoff und zwei Substanzen Gruppe (A) oder zwei Kohlenwasserstoffe und eine Substanz aus der Gruppe (A) aufweist.

8. Verwendung von einer Kombination aus mindestens einer Substanz aus der Gruppe (A) und mindestens einem Kohlenwasserstoff in Rasierhilfszubereitungen zur Verminderung des Verklebens von Rasierscherblättern, wobei Gruppe (A) folgende Substanzen umfasst: 1,3,4,6,7,8-Hexahydro-4,6,6,7,8,8-hexamethylcyclopenta-gamma-2-benzopyran, 2,6-Dimethyl-7-octen-2-ol, 2-Acetonapthone-1,2,3,4,5,6,7,8-octahydro-2,3,8,8-tetramethyl, 2-Isobutyl-4-hydroxy-4-methyltetrahydropyran, 2-tert-Pentylcyclohexylacetat, 3,7-Dimethyl-2,6-octadien-1-ol, 3-Methyl-5-phenyl-1-pentanol, 7-Acetyl-1,1,3,4,4,6-hexamethyltetralin, Adipinsäurediester, alpha-Amylcinnamaldehyd, alpha-Isomethylionon, Alpha-Methylionon, Amyl C Butylphenylmethylpropionalcinnamal, Amylcinnamylalkohol, Amylsalicylat, Anisalkohol, Benzoin, Benzylacetat, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citral, Citronellol, Citronellylmethylcrotonat, Citronenöl, Coumarin, Diethylsuccinat, d-Limonene, Ethylenbrassylate, Ethyllinalool, Eugenol, Evernia Furfuracea Extract, Evernia Prunastri Extract, Farnesol, Geraniol, Guajakholzöl, Heliotropin, Hexylcinnamal, Hexylsalicylat, Hydroxycitronellal, Hydroxyisohexyl 3-Cyclohexencarboxaldehyde, Isoeugenol, Lavendelöl, Lemonenöl, Limonen, Linalool, Linalylacetat, Mandarinenöl, Menthyl PCA, Methyl-2 Octynoat, Methylbenzoat, Methylcedrylketone, Methyldihydrojasmonate, Methylheptenon, Muskatnussöl, p-t-Butyl-alpha-methyldihydrocinnamicaldehyde, Rosmarinöl, süßes Orangenöl, Terpineol, Tonkabohnenöl, Triethylcitrat, Vanillin und wobei der Kohlenwasserstoff 8 bis 22 Kohlenstoffatome aufweist.

9. Verwendung nach Anspruch 8, **dadurch gekennzeichnet**, das mindestens ein Kohlenwasserstoff gradkettiger und/oder verzweigter Natur ist.

10. Verwendung nach Anspruch 9, **dadurch gekennzeichnet, dass** mindestens ein Kohlenwasserstoff 10 bis 20 Kohlenstoffatomen aufweist, insbesondere 12 bis 18 Kohlenstoffatomen, ganz besonders 13 bis 16 Kohlenstoffatomen.

11. Verwendung nach Anspruch 10, **dadurch gekennzeichnet, dass** als Kohlenwasserstoff Isohexadekan und/oder C13- C16 Isoparaffin gewählt wird.

12. Verwendung nach mindesten einem der Ansprüche 8 bis 11, **dadurch gekennzeichnet, dass** die Substanz der Gruppe (A) gewählt wird aus: Linalool, Limonen, Benzylsalicylat, Hydroxyisohexyl 3-Cyclohexencarboxaldehyde, Isoeugenol, Hydroxycitronellal, Coumarin, Eugenol, Citral, BHT, Citronellol, Geraniol,Benzylbenzoate, Cinnamal, Benzylalkohol, Farnesol, Cinnamylalkohol.

13. Verwendung nach mindesten einem der Ansprüche 8 bis 12, **dadurch gekennzeichnet, dass** die Substanz der Gruppe (A) Linalool ist.

14. Verwendung nach mindesten einem der Ansprüche 8 bis 12, **dadurch gekennzeichnet, dass** die Zubereitung einen Kohlenwasserstoff und zwei Substanzen Gruppe (A) oder zwei Kohlenwasserstoffe und eine Substanz aus der Gruppe (A) aufweist.
